# EUROPÄISCHE PATENTANMELDUNG

(11) **EP 3 909 629 A1**
(43) Veröffentlichungstag der Anmeldung: **17.11.2021**
(21) Anmeldenummer: 20195165.4
(22) Anmeldetag: 08.09.2020
(51) Int. Cl.: A61M 16/00, A61M 16/10

(54) **SYSTEM UND VERFAHREN ZUR FILTRATION VON ATEMGAS SOWIE PUFFERBEHÄLTER FÜR EIN SYSTEM ZUR FILTRATION VON ATEMGAS**

(71) Anmelder: ZeoSys Medical GmbH, 14943 Luckenwalde (DE)
(72) Erfinder: Friedrich, Thomas, 10367 Berlin (DE); Ewers, Christian, 14943 Luckenwalde (DE)
(74) Vertreter: Schulz Junghans Patentanwälte PartGmbB

(57) **Zusammenfassung**

Die Erfindung betrifft ein System (1) zur Filtration von Atemgas (2) eines Patienten, aufweisend einen Filter (20) zur Adsorption von Inhalationsanästhetika, wobei der Filter (20) einen Filtereinlass (21) und einen Filterauslass (22) aufweist, und einen Pufferbehälter (10) mit einem Pufferbehälterinnenraum (11), einem mit dem Filterauslass (22) verbundenen Pufferbehältereinlass (12) und einem Pufferbehälterauslass (13), wobei der Pufferbehälter (10) mindestens eine Lufteintrittsöffnung (14) aufweist, die den Pufferbehälterinnenraum (11) mit der Umgebung des Pufferbehälters (10) verbindet, so dass beim Absaugen des gefilterten Atemgases (3) aus dem Pufferbehälterinnenraum (11) über den Pufferbehälterauslass (13) Umgebungsluft (4) über die mindestens eine Lufteintrittsöffnung (14) in den Pufferbehälterinnenraum (11) einströmt.

Die Erfindung betrifft weiterhin den Pufferbehälter (10) für das System (1) sowie ein Verfahren zur Filtration von Atemgas (2) unter Verwendung des Systems (1).

## Beschreibung

Die Erfindung betrifft ein System zur Filtration von Atemgas eines Patienten sowie ein Verfahren zur Filtration von Atemgas mittels des Systems und einen Pufferbehälter für das System zur Filtration von Atemgas. Das System kann insbesondere dazu verwendet werden, Inhalationsanästhetika, insbesondere halogenierte Inhalationsanästhetika, aus der Ausatemluft eines sedierten oder narkotisierten Patienten zu entfernen und insbesondere in Verbindung mit einem in Krankenhäusern zum Einsatz kommenden Anästhesiegasfortleitungssystem (AGFS) verwendet werden. Der Patient ist dabei typischerweise an ein Beatmungssystem angeschlossen, welches dem Patienten das Inhalationsanästhetikum über die Atemwege zuführt.

Inhalationsanästhetika werden häufig verwendet, um Patienten z.B. während Operationen zu betäuben oder zu sedieren. Dabei kommen als Inhalationsanästhetika verbreitet halogenierte Kohlenwasserstoffverbindungen, insbesondere fluorierte Kohlenwasserstoffverbindungen (z.B. Flurane wie Sevofluran, Isofluran, Enfluran, Halothan und Desfluran oder ein Gemisch daraus) zum Einsatz.

Diese Stoffe sind zum einen bei längerer Exposition gesundheitsschädlich und wirken zum anderen ozonzersetzend. Daher ist das Freisetzen der ausgeatmeten Inhalationsanästhetika in die Umwelt ohne Filterung oder Absaugung aus Gründen des Gesundheits- und Umweltschutzes untragbar.

Aus dem Stand der Technik sind Filtermaterialien (z.B. auf Basis von Zeolithen und/oder Aktivkohle) bekannt, die in der Lage sind, solche Inhalationsanästhetika zu adsorbieren. Diese sind z.B. in den Dokumenten EP 2237854 A1, EP 2946826 A1, EP 2926897 A1 und WO 2007/093640 A1 beschrieben.

Weiterhin sind sogenannte Anästhesiegasfortleitungssysteme zum Absaugen von Anästhesiegasen aus Operationssälen bekannt.

Die oben beschriebenen Filtermaterialien sind prinzipiell geeignet, die Inhalationsanästhetika vollständig aus der Ausatemluft zu entfernen und sind darum sowie aufgrund ihres einfacheren Aufbaus und Betriebs ökologisch und ökonomisch vorteilhaft gegenüber reinen Absauganlagen.

Dennoch wäre die Kombination einer Inhalationsanästhetika-Filteranlage mit einer Absaugvorrichtung als zusätzliche Schutzmaßnahme wünschenswert, insbesondere um den Gesundheitsschutz des medizinischen Personals bei einem Defekt der Filteranlage bei gleichzeitigem Schutz der Atmosphäre vor der Emission von ozonschädigenden Treibhausgasen zu gewährleisten.

Wenn eine herkömmliche Filteranlage an ein vorhandenes Anästhesiegasfortleitungssystem angeschlossen wird, ergibt sich jedoch das Problem, dass durch den an der Ausgangsseite des Filters anliegenden Unterdruck eine Desorption der Inhalationsanästhetika stattfindet, wodurch das Filtermaterial entladen wird und ein Teil der Inhalationsanästhetika in die Umwelt entweicht. Zusätzlich verringert sich durch den über das Anästhesiegasfortleitungssystem erzeugten Unterdruck die Genauigkeit der Detektion von Inhalationsanästhetika durch Sensoren, was die Ermittlung der tatsächlichen Filterbeladung erschwert.

Somit ergibt sich die Aufgabe, ein System und ein Verfahren zur Filtration von Inhalationsanästhetika umfassendem Atemgas eines Patienten zur Verfügung zu stellen, welches im Hinblick auf die oben genannten Nachteile des Standes der Technik verbessert ist, insbesondere welches bei einer Kombination mit einem Anästhesiegasfortleitungssystem zu einer reduzierten Entladung des Filters und einer Erhöhung der Genauigkeit der Füllstandsmessung des Filters führt.

Diese Aufgabe wird durch den Gegenstand der unabhängigen Ansprüche 1 (Pufferbehälter), 3 (System zur Filtration von Atemgas) und 13 (Verfahren zur Filtration von Atemgas) gelöst. Vorteilhafte Ausgestaltungen der Erfindung sind in den Unteransprüchen 2, 4-12 und 14-15 angegeben und werden im Folgenden beschrieben.

Ein erster Aspekt der Erfindung betrifft einen Pufferbehälter für ein System zur Filtration von Atemgas eines Patienten, wobei der Pufferbehälter einen Pufferbehälterinnenraum, einen mit einem Filterauslass eines Filters des Systems verbindbaren, bzw. in Strömungsverbindung bringbaren, Pufferbehältereinlass zum Einleiten gefilterten Atemgases in den Pufferbehälterinnenraum und einen Pufferbehälterauslass zum Absaugen des gefilterten Atemgases aus dem Pufferbehälterinnenraum mittels eines Anästhesiegasfortleitungssystems aufweist. Erfindungsgemäß weist der Pufferbehälter mindestens eine Lufteintrittsöffnung, insbesondere mehrere Lufteintrittsöffnungen, auf, die den Pufferbehälterinnenraum mit der Umgebung des Pufferbehälters verbindet bzw. verbinden. Über die Lufteintrittsöffnung bzw. die Lufteintrittsöffnungen strömt beim Absaugen des gefilterten Atemgases aus dem Pufferbehälterinnenraum über den Pufferbehälterauslass Umgebungsluft in den Pufferbehälterinnenraum ein.

Somit wird der durch die Absaugung über das Anästhesiegasfortleitungssystem entstehende Unterdruck an der Ausgangsseite des Filters weitgehend reduziert oder sogar vollständig vermieden, so dass sich der Filter durch den zusätzlichen Absaugvorgang vorteilhafterweise weniger entlädt. Auf diese Weise lässt sich eine Filtrationsanlage auf einfache Weise mit einem Absaugsystem kombinieren, um eine zusätzliche Schutzwirkung vor austretenden Inhalationsanästhetika zu gewährleisten.

Gemäß einer Ausführungsform ist der Pufferbehälter entlang einer Längsachse erstreckt, wobei insbesondere die Längsachse in einer Durchströmungsrichtung des Filters verläuft, d.h. in der Richtung, in welcher das Atemgas den Filter des Systems gemäß dem zweiten Aspekt zwischen dem Filtereinlass und dem Filterauslass durchströmt.

Gemäß einer weiteren Ausführungsform weist der Pufferbehälter eine in Umfangsrichtung bezüglich der Längsachse verlaufende Pufferbehälterwandung auf, die den Pufferbehälterinnenraum umschließt.

Gemäß einer weiteren Ausführungsform ist die mindestens eine Lufteintrittsöffnung, insbesondere sind die Lufteintrittsöffnungen, in der Pufferbehälterwandung angeordnet.

Gemäß einer weiteren Ausführungsform ist der Pufferbehälterauslass in der Pufferbehälterwandung angeordnet.

Ein zweiter Aspekt der Erfindung betrifft ein System zur Filtration von Atemgas eines Patienten.

Das System weist einen Filter zur Adsorption von, insbesondere halogenierten, weiter insbesondere fluorierten, Inhalationsanästhetika auf, wobei der Filter einen Filtereinlass zum Einleiten von Atemgas eines Patienten in den Filter und einen Filterauslass aufweist, aus dem das gefilterte Atemgas austritt.

Weiterhin weist das System einen Pufferbehälter gemäß dem ersten Aspekt der Erfindung auf, also einen Pufferbehälter, der einen Pufferbehälterinnenraum, einen mit einem Filterauslass des Filters des Systems in Strömungsverbindung stehenden Pufferbehältereinlass zum Einleiten gefilterten Atemgases in den Pufferbehälterinnenraum und einen Pufferbehälterauslass zum Absaugen des gefilterten Atemgases aus dem Pufferbehälterinnenraum mittels eines Anästhesiegasfortleitungssystems aufweist, wobei der Pufferbehälter mindestens eine Lufteintrittsöffnung aufweist, die den Pufferbehälterinnenraum mit der Umgebung des Pufferbehälters verbindet, so dass beim Absaugen des gefilterten Atemgases aus dem Pufferbehälterinnenraum über den Pufferbehälterauslass Umgebungsluft über die mindestens eine Lufteintrittsöffnung in den Pufferbehälterinnenraum einströmt.

Der Pufferbehältereinlass des Pufferbehälters steht also mit dem Filterauslass des Filters des Systems in Strömungsverbindung, so dass mittels des Filters gefiltertes Atemgas über den Pufferbehältereinlass in den Pufferbehälterinnenraum einleitbar ist. Dabei muss die Strömungsverbindung nicht zwingend gegen die Umgebung abgedichtet sein. Es ist z.B. möglich, dass der Filter lose in einen Filteraufnahmebehälter hineingestellt ist, wobei der Filteraufnahmebehälter mit dem Pufferbehältereinlass, insbesondere dichtend, verbunden ist.

Durch den erfindungsgemäßen Pufferbehälter wird der durch die Absaugung über das Anästhesiegasfortleitungssystem entstehende Unterdruck an der Ausgangsseite des Filters weitgehend reduziert oder sogar vollständig vermieden, so dass sich der Filter durch den zusätzlichen Absaugvorgang vorteilhafterweise weniger entlädt. Dabei wird der entstehende Unterdruck insbesondere soweit reduziert, dass die vom Patienten kommende Ausatemluft, bzw. der Strom der Ausatemluft, so gering wie möglich beeinflusst wird, wobei auf Grundlage des sich dadurch einstellenden Gleichgewichts von Adsorptions- und Desorptionsprozessen eine maximal mögliche Adsorptionskapazität des Filters möglich ist. Auf diese Weise lässt sich eine Filtrationsanlage auf einfache Weise mit einem Absaugsystem kombinieren, um eine zusätzliche Schutzwirkung vor austretenden Inhalationsanästhetika zu gewährleisten.

Gemäß einer Ausführungsform weist das System einen Filteraufnahmebehälter zur Aufnahme des Filters auf.

Gemäß einer weiteren Ausführungsform weist der Filteraufnahmebehälter einen Verbindungsabschnitt zum Verbinden mit dem Pufferbehälter auf, wobei der Verbindungsabschnitt einen Verbindungsabschnittsinnenraum aufweist, der mit dem Filterauslass des Filters in Strömungsverbindung steht.

Alternativ dazu kann der Pufferbehälter auch integral mit dem Filteraufnahmebehälter ausgeformt sein, also mit dem Filteraufnahmebehälter ein einstückiges zusammenhängendes Bauteil bilden.

Gemäß einer weiteren Ausführungsform ist der Pufferbehälter über eine lösbare Verbindung, insbesondere eine Steckverbindung, mit dem Filteraufnahmebehälter, insbesondere dem Verbindungsabschnitt, verbunden. Dies hat insbesondere den Vorteil, dass sich der Pufferbehälter leicht austauschen und reinigen lässt.

Gemäß einer weiteren Ausführungsform weist das System eine Dichtung auf, wobei die Dichtung die lösbare Verbindung zwischen dem Pufferbehälter und dem Filteraufnahmebehälter, insbesondere dem Verbindungsabschnitt, abdichtet.

Gemäß einer weiteren Ausführungsform weist das System eine austauschbare Filterkartusche auf, wobei die Filterkartusche den Filter aufweist, und wobei die Filterkartusche in dem Filteraufnahmebehälter angeordnet ist. Somit lässt sich der Filter nach vollständiger Befüllung auf einfache Weise tauschen und regenerieren.

Gemäß einer weiteren Ausführungsform weist der Verbindungsabschnitt ein mit dem Verbindungsabschnittsinnenraum in Strömungsverbindung stehendes Verbindungsrohr mit einer Ausströmöffnung auf, wobei das Verbindungsrohr in den Pufferbehälterinnenraum des Pufferbehälters hineinragt, so dass die Ausströmöffnung in dem Pufferbehälterinnenraum angeordnet ist, wenn der Verbindungsabschnitt mit dem Pufferbehälter verbunden ist.

Gemäß einer weiteren Ausführungsform ist das Verbindungsrohr von dem Pufferbehälterauslass beabstandet, so dass die Ausströmöffnung nicht direkt in den Pufferbehälterauslass sondern in den Pufferbehälterinnenraum mündet. Das Verbindungsrohr ist also offen zum Pufferbehälterinnenraum.

Das Verbindungsrohr ist insbesondere so konfiguriert, dass eine relativ kleine Ausströmöffnung gebildet wird, die einen maximalen Abstand von den Lufteintrittsöffnungen des Pufferbehälters hat, um das aus dem Verbindungsrohr austretende gereinigte Atemgas indirekt über die durch die Lufteintrittsöffnungen einströmende Luft über den an dem Pufferbehälterauslass angeschlossenen Schlauch in das AGFS abzuleiten. Auf diese Weise wird weitgehend verhindert, dass gefiltertes Atemgas, was bei einer defekten Filtereinheit möglicherweise noch Reste an Inhalationsanästhetika aufweist, über die Lufteintrittsöffnungen aus dem Pufferbehälterinnenraum austritt. Das Verhältnis der Querschnitte der Ein- und Austrittsöffnungen sollte dabei insbesondere so gewählt werden, dass bei einer defekten oder nicht rechtzeitig getauschten vollen Filtereinheit kein gefiltertes Atemgas mit Inhalationsanästhetika über die Lufteintrittsöffnungen aus dem Pufferbehälterinnenraum austritt.

Gemäß einer weiteren Ausführungsform verjüngt sich das Verbindungsrohr in Richtung von dem Verbindungsabschnittsinnenraum zu der Ausströmöffnung hin, d.h. eine senkrecht zu einer Erstreckungsrichtung des Verbindungsrohres verlaufende Querschnittsfläche des Verbindungsrohres wird in Richtung von dem Verbindungsabschnittsinnenraum zu der Ausströmöffnung hin geringer. Alternativ dazu kann das Verbindungsrohr auch gerade verlaufen und/oder über seine Länge einen konstanten Innendurchmesser aufweisen.

Gemäß einer weiteren Ausführungsform mündet der Pufferbehälterauslass in einen Pufferbehälterauslassstutzen, insbesondere wobei sich der Pufferbehälterauslassstutzen in Richtung von dem Pufferbehälterinnenraum zum Anästhesiegasfortleitungssystem verjüngt. Dadurch wird die Strömungsgeschwindigkeit innerhalb des Pufferbehälters minimiert.

Gemäß einer weiteren Ausführungsform weist das System einen Gassensor zur, insbesondere quantitativen, Detektion einer Gasspezies, insbesondere Inhalationsanästhetika, weiter insbesondere fluorierte Inhalationsanästhetika, weiter insbesondere fluorierte Inhalationsanästhetika, in dem gefilterten Atemgas auf. Dabei kann die Gasspezies insbesondere das Inhalationsanästhetikum selbst oder ein Abbauprodukt des Inhalationsanästhetikums sein.

Gemäß einer weiteren Ausführungsform ist der Gassensor in dem Verbindungsabschnittsinnenraum des Verbindungsabschnitts des Filteraufnahmebehälters angeordnet.

Gemäß einer weiteren Ausführungsform weist das System eine mit dem Gassensor gekoppelte Hinweisvorrichtung auf, die dazu ausgebildet ist, einen Hinweis zu erzeugen, wenn die Gasspezies von dem Gassensor in dem gefilterten Atemgas detektiert wird.

Dabei bedeutet "gekoppelt", dass die Hinweisvorrichtung in der Weise mit dem Gassensor wirkverbunden ist, dass die Hinweisvorrichtung bei einem vorgegebenen Messwert des Gassensors einen entsprechenden Hinweis erzeugt. Die Hinweisvorrichtung kann direkt oder indirekt (z.B. über eine Recheneinheit) mit dem Gassensor gekoppelt sein. Die Kopplung kann z.B. über eine elektrische Verbindung oder eine Datenverbindung realisiert sein, insbesondere so, dass die Hinweisvorrichtung direkt oder indirekt über die Recheneinheit ein Eingangssignal des Gassensors empfängt.

Bei dem Hinweis kann es sich insbesondere um einen optischen Hinweis (z.B. das Aufleuchten eines Leuchtmittels wie einer LED oder um einen akustischen Hinweis (z.B. einen Warnton) handeln.

Gemäß einer weiteren Ausführungsform ist Gassensor dazu ausgebildet, eine Konzentration oder eine Menge der Gasspezies in dem gefilterten Atemgas zu detektieren, wobei die Hinweisvorrichtung dazu ausgebildet ist, den Hinweis zu erzeugen, wenn der Gassensor eine voreingestellte Mindestkonzentration oder eine voreingestellte Mindestmenge der Gasspezies in dem gefilterten Atemgas detektiert.

Der durch die Hinweisvorrichtung ausgegebene Hinweis zeigt insbesondere an, dass der maximale Füllstand, d.h. insbesondere die maximale Belegung des Filters mit Inhalationsanästhetika oder die maximale Aufnahmekapazität des Filters erreicht ist bzw. bei gleichbleibendem Betrieb bald erreicht sein wird.

Ein dritter Aspekt der Erfindung betrifft ein Verfahren zur Filtration von Atemgas eines Patienten, aufweisend die folgenden Schritte:
- Bereitstellen eines Systems nach dem zweiten Aspekt der Erfindung, und
- Anschließen des Pufferbehälterauslasses des Pufferbehälters des Systems an ein Anästhesiegasfortleitungssystem, und
- Einleiten des Atemgases des Patienten in den Filter des Systems über den Filtereinlass, wobei das Atemgas ein Inhalationsanästhetikum aufweist, und
- Absaugen des mittels des Filters gefilterten Atemgases aus dem Pufferbehälterinnenraum des Pufferbehälters mittels des Anästhesiegasfortleitungssystem.

Gemäß einer Ausführungsform des Verfahrens weist das Inhalationsanästhetikum eine halogenierte Kohlenwasserstoffverbindung, insbesondere eine fluorierte Kohlenwasserstoffverbindung, weiter insbesondere ein Fluran, auf.

Gemäß einer weiteren Ausführungsform ist das Inhalationsanästhetikum ausgewählt aus der Gruppe aufweisend Sevofluran, Isofluran, Enfluran, Halothan und Desfluran oder einem Gemisch daraus.

Gemäß einer Ausführungsform des Verfahrens wird mittels eines Gassensors eine Gasspezies, insbesondere das Inhalationsanästhetikum, in dem gefilterten Atemgas detektiert. Dabei kann die Gasspezies insbesondere das Inhalationsanästhetikum selbst oder ein Abbauprodukt des Inhalationsanästhetikums sein.

Gemäß einer weiteren Ausführungsform des Verfahrens wird mittels einer mit dem Gassensor gekoppelten Hinweisvorrichtung ein, insbesondere optischer oder akustischer, Hinweis erzeugt, wenn die Gasspezies von dem Gassensor in dem gefilterten Atemgas detektiert wird.

Soweit Alternativen einzelner trennbarer Merkmale in dieser Spezifikation als Ausführungsformen angegeben sind, versteht sich, dass die Alternativen frei kombinierbar sind, um weitere Ausführungsformen der hier offenbarten Erfindung zu bilden.

Weitere Einzelheiten und Vorteile der Erfindung werden durch die nachfolgende Beschreibung von Ausführungsbeispielen anhand von Figuren erläutert. Diese Ausführungsbeispiele sind nicht als beschränkend für den Schutzbereich der Erfindung zu verstehen.

Es zeigen:
- Fig. 1: eine schematische Darstellung eines Systems zur Filtration von Atemgas eines Patienten gemäß einer Ausführungsform der Erfindung;
- Fig. 2: eine Schnittdarstellung eines Pufferbehälters gemäß einer Ausführungsform der Erfindung im Querschnitt bezüglich der Längsachse;
- Fig. 3: eine Seitenansicht des in Fig. 2 gezeigten Pufferbehälters;
- Fig. 4: eine Seitenansicht eines Filteraufnahmebehälters gemäß einer Ausführungsform der Erfindung.

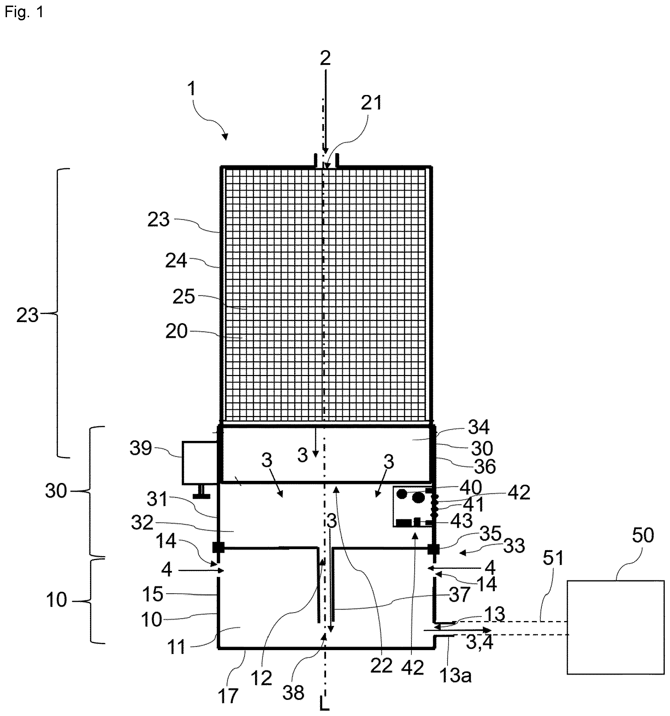

Die Figur 1 zeigt eine schematische Darstellung eines Systems 1 zur Filtration von Atemgas 2 eines Patienten gemäß einer Ausführungsform der vorliegenden Erfindung.

Das System 1 weist einen Pufferbehälter 10 sowie ein mit dem Pufferbehälter 10 verbundenen Filteraufnahmebehälter 30 auf, in den eine Filterkartusche 23 eingesetzt ist, wobei die Filterkartusche 23 einen Filter 20 enthält.

Das zusammengesetzte System 1 ist in dem in Figur 1 gezeigten Beispiel zylinderförmig ausgebildet und entlang der Längsachse L erstreckt. Diese Längsachse L ist im betriebsbereiten Zustand des Systems 1 insbesondere vertikal ausgerichtet. In einer solchen vertikalen Anordnung bildet somit die Filterkartusche 23 mit dem Filter 20 das obere Ende des Systems 1 und der Pufferbehälter 10 bildet das untere Ende des Systems 1.

Die Filterkartusche 23 weist an ihrem oberen Ende einen Filtereinlass 21 zum Einleiten des Atemgases 2 eines Patienten auf, z.B. durch Anschließen eines Exspirationsventils eines Beatmungsgeräts an den Filtereinlass 21 über einen Schlauch.

Das Atemgas 2 enthält gasförmige Inhalationsanästhetika, z.B. Sevofluran oder Desfluran.

Die Filterkartusche 23 weist eine Filterkartuschenwandung 24 auf, die einen Raum umschließt, in dem ein Filtermaterial 25 angeordnet ist. Das Filtermaterial 25 ist dazu geeignet, insbesondere halogenierte, Inhalationsanästhetika zu adsorbieren. Zu diesem Zweck kann als Filtermaterial 25 z.B. ein Schüttgut oder Granulat aus Zeolith und/oder Aktivkohle aufweisen, insbesondere aus hydrophobem Zeolith oder hydrophober Aktivkohle.

Wenn das Atemgas 2 das Filtermaterial 25 durchströmt, werden die gasförmigen Inhalationsanästhetika an dem Filtermaterial 25 adsorbiert und somit aus dem Atemgas 2, insbesondere vollständig oder nahezu vollständig, entfernt, wodurch ein gefiltertes Atemgas 3 entsteht.

Das gefilterte Atemgas 3 verlässt den Filter 20 bzw. die Filterkartusche 23 über einen Filterauslass 22 und strömt im vorliegenden Beispiel über einen Verbindungsabschnitt 31 eines Filteraufnahmebehälters 30, in dem die Filterkartusche 23 angeordnet ist, und über einen Pufferbehältereinlass 12 in den Pufferbehälter 10 ein.

Um zu verhindern, dass etwaige Restbestände an Inhalationsanästhetika, die sich z.B. aufgrund eines defekten Filters 20 in dem gefilterten Atemgas 3 befinden könnten, in die Umgebung austreten, wird ein Pufferbehälter 10 über den Pufferbehälterauslass 13 mit einem Anästhesiegasfortleitungssystem 50 strömungstechnisch verbunden, sodass das gefilterte Atemgas 3 mittels des Anästhesiegasfortleitungssystem 50 aus dem Pufferbehälterinnenraum 11 des Pufferbehälters 10 abgesaugt werden kann.

Das Anästhesiegasfortleitungssystem 50 weist insbesondere eine Pumpe zum Erzeugen von Unterdruck auf (in Fig. 1 nicht gezeigt), welche z.B. über eine Leitung 51 mit einem Pufferbehälterauslassstutzen 13a verbunden werden kann, welcher den Pufferbehälterauslass 13 bildet.

Der Pufferbehälter 10 weist in seiner Pufferbehälterwandung 15, die den Pufferbehälter 11 umschließt, mindestens eine Lufteintrittsöffnung 14, insbesondere mehrere Lufteintrittsöffnungen 14, auf. Diese Lufteintrittsöffnungen 14 bewirken, dass sich beim Absaugen des gefilterten Atemgases 3 über das Anästhesiegasfortleitungssystem 50 kein Unterdruck (bzw. ein geringerer Unterdruck im Vergleich zu einem geschlossenen Pufferbehälterinnenraum 11) in dem Pufferbehälterinnenraum 11 aufbauen kann, da Umgebungsluft 4 aus der Umgebung des Systems 1 über die Lufteintrittsöffnungen 14 in den Pufferbehälterinnenraum 11 nachströmt. Durch den geringeren Unterdruck wird der Filter 20 durch die Absaugung über das Anästhesiegasfortleitungssystem vorteilhafterweise weniger stark entladen und der Atemluftstrom des Patienten wird weniger stark beeinflusst.

Die nachströmende Umgebungsluft 4 mischt sich mit dem gefilterten Atemgas 3, wobei die Mischung aus dem gefilterten Atemgas 3 und der Umgebungsluft 4 mittels des Anästhesiegasfortleitungssystems 50 aus dem Pufferbehälter 10 abgezogen wird.

In dem in Figur 1 gezeigten Beispiel ist die Filterkartusche 23 über eine Öffnung am oberen Ende des Filteraufnahmebehälters 30 teilweise in den Filteraufnahmebehälterinnenraum 34 eingesetzt und das obere Ende der Filterkartusche 23 ragt aus dem Filteraufnahmebehälter 30 heraus.

Der Pufferbehälter 10 ist in dem in Figur 1 gezeigten Beispiel über eine lösbare Steckverbindung 33 mit einem Verbindungsabschnitt 31 des Filteraufnahmebehälters 30 verbunden. Optional kann die Steckverbindung z.B. mit einer Arretierschraube gesichert werden, um ein unerwünschtes Lösen des Pufferbehälters 10 von dem Verbindungabschnitt 31 des Filteraufnahmebehälters 30 zu verhindern. Eine Dichtung 35, z.B. eine Dichtlippe aus Silikon oder einem anderen geeigneten Material, dichtet die Verbindung 33 zwischen dem Pufferbehälter 10 und dem Verbindungsabschnitt 31 des Filteraufnahmebehälters 30 ab.

Der Verbindungsabschnitt 31 weist einen von einer Wandung umschlossenen Verbindungsabschnittsinnenraum 32 und ein von dem Verbindungsabschnittsinnenraum 32 abzweigendes Verbindungsrohr 37 mit einer Ausströmöffnung 38 auf. Wenn der Pufferbehälter 10 mit dem Verbindungsabschnitt 31 verbunden ist, ragt das Verbindungsrohr 37 in den Pufferbehälter 11 hinein. Die Ausströmöffnung 38 ist dabei von dem Pufferbehälterauslass 13 beanstandet, sodass das gefilterte Atemgas 3 nicht direkt aus dem Verbindungsabschnittsinnenraum 32 über das Anästhesiegasfortleitungssystem 50 abgesaugt wird, sondern in den Pufferbehälterinnenraum 11 eintritt.

Das Verbindungsrohr 37 verläuft dabei in vertikaler Richtung, d. h. der Gasstrom des gefilterten Atemgases 3 durchströmt das Verbindungsrohr 37 in Richtung einer stirnseitigen Wandung 17 des Pufferbehälters 10.

Die beschriebene Anordnung und Ausformung des Verbindungsrohres 37 hat zur Folge, dass das gefilterte Atemgas 3 über eine relativ kleine und relativ weit von den Lufteintrittsöffnungen 14 entfernte Ausströmöffnung 38 in den Pufferbehälter 10 hineinströmt, was eventuelle Verwirbelungen minimiert, die zum Austreten von gefiltertem Atemgas 3 über die Lufteintrittsöffnungen 14 in die Umgebung führen könnten. Ein solches Austreten in die Umgebung wäre problematisch, falls sich z.B. bei einer Überschreitung des maximalen Füllgrads des Filters 20 Reste des Inhalationsanästhetikum in dem gefilterten Atemgas 3 befinden sollten.

Zur indirekten Kontrolle des Füllstands des Filters 20, also der Menge von an dem Filtermaterial 25 adsorbierten Inhalationsanästhetika, weist das System 1 einen Gassensor 40 auf, der dazu ausgebildet ist, gasförmige Inhalationsanästhetika in dem gefilterten Atemgas 3 zu detektieren. Eine solche indirekte Füllstandsmessung ist möglich, weil ein hoher Füllgrad des Filters zu einem sinkenden Adsorptionsvermögen des Filters 20 und somit zu einer unvollständigen Filterung des Atemgases 2 führt. Somit sind bei einem zu hohen Füllgrad mittels des Gassensors 40 Reste des Inhalationsanästhetikums in dem gefilterten Atemgas 3 nachweisbar. Der Gassensor 40 kann z.B. als VOC (volatile organic compound)- Sensor ausgebildet sein, wobei das Messprinzip insbesondere auf der veränderten Leitfähigkeit von dotierten Metalloxiden in Gegenwart von organischen Verbindungen beruht.

Der erfindungsgemäße Pufferbehälter 10 mit mindestens einer Lufteintrittsöffnung 14 hat den zusätzlichen Effekt, dass aufgrund des geringeren Unterdrucks eine genauere Bestimmung der Menge an Inhalationsanästhetika in dem gefilterten Atemgas 3 mittels des Gassensors 40 möglich ist.

In dem in Figur 1 gezeigten Beispiel ist der Gassensor 40 innerhalb einer Elektronikeinheit 42 angeordnet, wobei sich die Elektronikeinheit 42 in dem Verbindungsabschnittsinnenraum 32 des Verbindungsabschnitts 31 des Filteraufnahmebehälters 30 befindet. Die Elektronikeinheit 42 weist weiterhin eine mit dem Gassensor 40 wirkverbundene Recheneinheit 43 und eine Hinweisvorrichtung 41 zur Ausgabe eines, insbesondere optischen oder akustischen, Hinweises auf.

Der Gassensor 40 generiert insbesondere ein Signal, das von der Menge oder Konzentration der in dem gefilterten Atemgas 3 enthaltenen Inhalationsanästhetika abhängig ist. Dieses Signal wird insbesondere an die Recheneinheit 43 übermittelt und die Recheneinheit 43 ermittelt mithilfe von Hardwarekomponenten oder Softwarekomponenten, ob eine voreingestellte Grenzkonzentration an Inhalationsanästhetika in dem gefilterten Atemgas 3 erreicht oder überschritten ist. Wenn dies der Fall ist, steuert die Recheneinheit 43 die Hinweisvorrichtung 41 an, woraufhin diese einen Hinweis erzeugt, dass der Füllstand des Filters 20 einen kritischen Wert erreicht hat bzw. dass die maximale Kapazität des Filters 20 erreicht ist.

Beispielsweise kann die Hinweisvorrichtung 41 mindestens ein Leuchtmittel, z.B. eine LED, aufweisen, wobei ein Leuchten des Leuchtmittels als optischer Hinweis dient, dass z.B. ein kritischer Füllstand des Filters 20 erreicht ist. Optional kann die Hinweisvorrichtung auch mehrere, z.B. verschiedenfarbige Leuchtmittel wie LEDs aufweisen, wobei das Aufleuchten der einzelnen Leuchtmittel z.B. unterschiedliche Konzentrationen des Inhalationsanästhetikum in dem gefilterten Atemgas 3 und somit unterschiedliche Füllstände des Filters anzeigt. Beispielsweise könnte das Leuchten einer ersten gelben LED anzeigen, dass der maximale Füllstand des Filters 20 bald erreicht ist, wohingegen das Leuchten einer zweiten roten LED einen vollständig gefüllten Filter 20 anzeigt, der sofort ausgetauscht werden muss.

Alternativ oder zusätzlich zu einem optischen Hinweis kann die Hinweisvorrichtung 41 z.B. auch einen akustischen Hinweis, wie z.B. einen Warnton, ausgeben, wenn eine vorgegebene Menge des Inhalationsanästhetikums in dem gefilterten Atemgas mittels des Gassensors 40 detektiert wird.

Figur 1 zeigt weiterhin eine außen an dem Filteraufnahmebehälter 30 angeordnete Haltevorrichtung 39, die z.B. als Schraubklemme ausgeformt sein kann. Die Haltevorrichtung 39 dient dazu, das System 1 z.B. an einem Anästhesiewagen sicher zu befestigen.

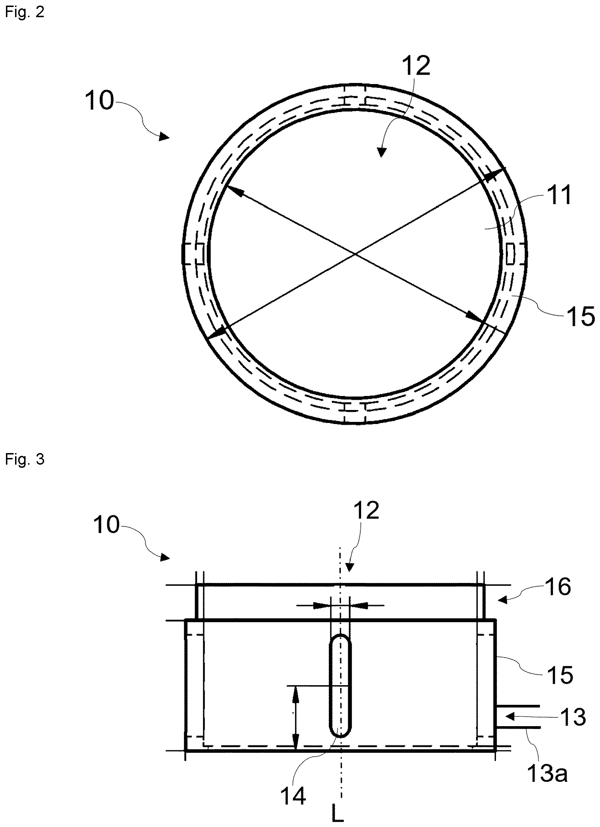

Die Figuren 2 und 3 zeigen eine spezielle Ausgestaltung des Pufferbehälters 10 nach einer Ausführungsform der Erfindung, wobei die Figur 2 einen Querschnitt senkrecht zu der Längsachse L darstellt und die Figur 3 den Pufferbehälter 10 in einer Seitenansicht zeigt.

Aus der Figur 2 ist ersichtlich, dass der Pufferbehälter 10 kreiszylinderförmig ist, wobei der Pufferbehälterinnenraum 12 ebenfalls einen kreisförmigen Querschnitt aufweist.

Die Figur 3 zeigt im Detail eine der in der Pufferbehälterwandung 15 angeordneten Lufteintrittsöffnungen 14, welche in diesem Beispiel als entlang der Längsachse L erstrecktes abgerundetes Langloch ausgeformt ist.

Weiterhin ist in Figur 3 gezeigt, dass die Pufferbehälterwandung 15 an ihrem dem Pufferbehältereinlass 12 zugewandten Seite (in Figur 3 oben) eine in Umfangsrichtung bezüglich der Längsachse L umlaufende Aussparung 16 aufweist, sodass im Bereich der Aussparung 16 senkrecht zu der Längsachse L die Wandungsdicke der Pufferbehälterwandung 15 reduziert ist. Diese reduzierte Wandungsdicke korrespondiert mit dem Innendurchmesser des Verbindungsabschnittsinnenraums 32 des Verbindungsabschnitts 31 des Filteraufnahmebehälters 30, um die Steckverbindung zwischen dem Filteraufnahmebehälter 30 und dem Pufferbehälter 10 zu realisieren.

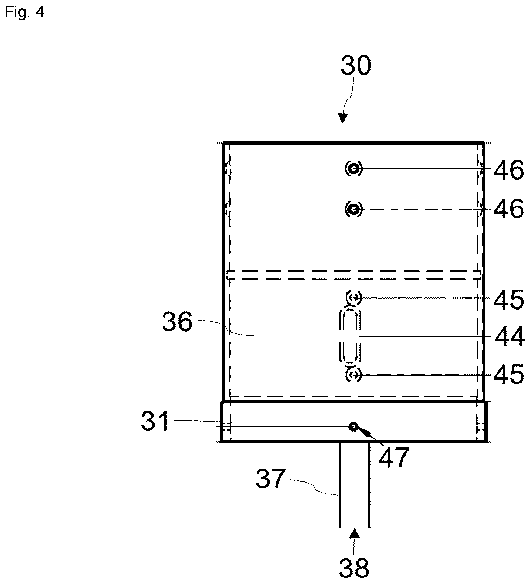

Schließlich zeigt die Figur 4 eine Seitenansicht des Filteraufnahmebehälters 30.

Weiterhin ist ein in der Filteraufnahmebehälterwandung 36 angeordnetes Langloch 44 zum Positionieren der Hinweisvorrichtung 41 (siehe Figur 1) in Form einer LED-Anordnung gezeigt. In ersten Durchgangsöffnungen 45 in der Filteraufnahmebehälterwandung 36 unterhalb und oberhalb des Langlochs 44 können z.B. Schrauben zur Fixierung der Hinweisvorrichtung 41 aufgenommen werden.

Zweite Durchgangsöffnungen 46 am oberen Ende des Filteraufnahmebehälters 30 dienen insbesondere zur Aufnahme von Schrauben, um die Haltevorrichtung 39 (insbesondere Schraubklemme) an dem Filteraufnahmebehälter 30 zu befestigen.

Schließlich ist an dem Verbindungsabschnitt 31 eine dritte Durchgangsöffnung 47 vorgesehen, um eine Arretierschraube zum Sichern der Steckverbindung zwischen dem Filteraufnahmebehälter 30 und dem Pufferbehälter 10 aufzunehmen.

Der Verbindungsabschnitt 31 weist weiterhin ein Verbindungsrohr 37 mit einer Ausströmöffnung 38 auf, durch die das gefilterte Atemgas 3 in den Pufferbehälterinnenraum 11 einströmen kann, wenn der Pufferbehälter 10 mit dem Filteraufnahmebehälter 30 verbunden ist.

**Bezugszeichenliste**

| | |
|---|---|
| System zur Filtration von Atemgas | 1 |
| Atemgas | 2 |
| Gefiltertes Atemgas | 3 |
| Umqebunqsluft | 4 |
| Pufferbehälter | 10 |
| Pufferbehälterinnenraum | 11 |
| Pufferbehältereinlass | 12 |
| Pufferbehälterauslass | 13 |
| Pufferbehälterauslassstutzen | 13a |
| Lufteintrittsöffnung | 14 |
| Pufferbehälterwandunq | 15 |
| Vertiefung | 16 |
| Stirnseitiqe Wandung | 17 |
| Filter | 20 |
| Filtereinlass | 21 |
| Filterauslass | 22 |
| Filterkartusche | 23 |
| Filterkartuschenwandunq | 24 |
| Filtermaterial | 25 |
| Filteraufnahmebehälter | 30 |
| Verbindungsabschnitt | 31 |
| Verbindungsabschnittsinnenraum | 32 |
| Lösbare Verbindung | 33 |
| Filteraufnahmebehälterinnenraum | 34 |
| Dichtung | 35 |
| Filteraufnahmebehälterwandung | 36 |
| Verbindungsrohr | 37 |
| Ausströmöffnung | 38 |
| Haltevorrichtung | 39 |
| Gassensor | 40 |
| Hinweisvorrichtung | 41 |
| Elektronikeinheit | 42 |
| Recheneinheit | 43 |
| Langloch | 44 |
| Erste Durchqanqsöffnunq | 45 |
| Zweite Durchgangsöffnung | 46 |
| Dritte Durchqanqsöffnunq | 47 |
| Anästhesiegasfortleitungssystem | 50 |
| Anästhesieqasleitunq | 51 |
| Längsachse | L |

## Patentansprüche

1. Pufferbehälter (10) für ein System (1) zur Filtration von Atemgas (2) eines Patienten, wobei der Pufferbehälter (10)
- einen Pufferbehälterinnenraum (11),
- einen mit einem Filterauslass (22) eines Filters (20) des Systems (1) verbindbaren Pufferbehältereinlass (12) zum Einleiten gefilterten Atemgases (3) in den Pufferbehälterinnenraum (11) und
- einen Pufferbehälterauslass (13) zum Absaugen des gefilterten Atemgases (3) aus dem Pufferbehälterinnenraum (11) mittels eines Anästhesiegasfortleitungssystems (50)
aufweist, wobei der Pufferbehälter (10) mindestens eine Lufteintrittsöffnung (14) aufweist, die den Pufferbehälterinnenraum (11) mit der Umgebung des Pufferbehälters (10) verbindet, so dass beim Absaugen des gefilterten Atemgases (3) aus dem Pufferbehälterinnenraum (11) über den Pufferbehälterauslass (13) Umgebungsluft (4) über die mindestens eine Lufteintrittsöffnung (14) in den Pufferbehälterinnenraum (11) einströmt.

2. Pufferbehälter (10) nach Anspruch 1, **dadurch gekennzeichnet, dass** der Pufferbehälter (10) entlang einer Längsachse (L) erstreckt ist, wobei der Pufferbehälter (10) eine in Umfangsrichtung bezüglich der Längsachse (L) verlaufende Pufferbehälterwandung (15) aufweist, die den Pufferbehälterinnenraum (11) umschließt, wobei die mindestens eine Lufteintrittsöffnung (14) in der Pufferbehälterwandung (15) angeordnet ist, wobei insbesondere auch der Pufferbehälterauslass (13) in der Pufferbehälterwandung (15) angeordnet ist.

3. System (1) zur Filtration von Atemgas (2) eines Patienten, aufweisend
a. einen Filter (20) zur Adsorption von Inhalationsanästhetika, wobei der Filter (20) einen Filtereinlass (21) und einen Filterauslass (22) aufweist, und
b. einen Pufferbehälter (10) gemäß Anspruch 1 oder 2, wobei der Pufferbehältereinlass (12) des Pufferbehälters mit dem Filterauslass (22) des Filters (20) des Systems (1) in Strömungsverbindung steht, so dass mittels des Filters (20) gefiltertes Atemgas (3) über den Pufferbehältereinlass (12) in den Pufferbehälterinnenraum (11) einleitbar ist.

4. System (1) nach Anspruch 3, **dadurch gekennzeichnet, dass** das System (1) einen Filteraufnahmebehälter (30) zur Aufnahme des Filters (20) aufweist, wobei der Filteraufnahmebehälter (30) einen Verbindungsabschnitt (31) zum Verbinden mit dem Pufferbehälter (10) aufweist, wobei der Verbindungsabschnitt (31) einen Verbindungsabschnittsinnenraum (32) aufweist, der mit dem Filterauslass (22) des Filters (22) in Strömungsverbindung steht.

5. System (1) nach Anspruch 3 oder 4, **dadurch gekennzeichnet, dass** der Pufferbehälter (10) über eine lösbare Verbindung (33), insbesondere eine Steckverbindung, mit dem Filteraufnahmebehälter (30), insbesondere dem Verbindungsabschnitt (31) verbunden ist.

6. System (1) nach Anspruch 5, **dadurch gekennzeichnet, dass** das System (1) eine Dichtung (35) aufweist, wobei die Dichtung (35) die lösbare Verbindung (33) zwischen dem Pufferbehälter (10) und dem Filteraufnahmebehälter (30) abdichtet.

7. System (1) nach einem der Ansprüche 4 bis 6, **dadurch gekennzeichnet, dass** das System (1) eine austauschbare Filterkartusche (23) aufweist, wobei die Filterkartusche (23) den Filter (20) aufweist, und wobei die Filterkartusche (23) in dem Filteraufnahmebehälter (30) angeordnet ist.

8. System (1) nach einem der Ansprüche 4 bis 7, **dadurch gekennzeichnet, dass** der Verbindungsabschnitt (31) ein mit dem Verbindungsabschnittsinnenraum (32) in Strömungsverbindung stehendes Verbindungsrohr (37) mit einer Ausströmöffnung (38) aufweist, wobei das Verbindungsrohr (37) in den Pufferbehälterinnenraum (11) des Pufferbehälters (10) hineinragt.

9. System (1) nach Anspruch 8, **dadurch gekennzeichnet, dass** das Verbindungsrohr (37) von dem Pufferbehälterauslass (13) beabstandet ist.

10. System nach Anspruch 8 oder 9, **dadurch gekennzeichnet, dass** sich das Verbindungsrohr (37) in Richtung von dem Verbindungsabschnittsinnenraum (32) zu der Ausströmöffnung (38) hin verjüngt.

11. System (1) nach einem der Ansprüche 3 bis 10, **dadurch gekennzeichnet, dass** das System einen Gassensor (40) zur Detektion einer Gasspezies in dem gefilterten Atemgas aufweist, wobei insbesondere der Gassensor (40) in dem Verbindungsabschnittsinnenraum (31) angeordnet ist.

12. System (1) nach Anspruch 11, **dadurch gekennzeichnet, dass** das System eine mit dem Gassensor gekoppelte Hinweisvorrichtung (41) aufweist, die dazu ausgebildet ist, einen Hinweis zu erzeugen, wenn die Gasspezies von dem Gassensor (40) in dem gefilterten Atemgas (3) detektiert wird, wobei insbesondere der Gassensor (40) dazu ausgebildet ist, eine Konzentration oder eine Menge der Gasspezies in dem gefilterten Atemgas (3) zu detektieren, wobei die Hinweisvorrichtung (41) dazu ausgebildet ist, den Hinweis zu erzeugen, wenn der Gassensor (40) eine voreingestellte Mindestkonzentration oder eine voreingestellte Mindestmenge der Gasspezies in dem gefilterten Atemgas (3) detektiert.

13. Verfahren zur Filtration von Atemgas (2) eines Patienten, aufweisend die folgenden Schritte:
a. Bereitstellen eines Systems (1) nach einem der Ansprüche 3 bis 12,
b. Anschließen des Pufferbehälterauslasses (13) des Pufferbehälters (10) des Systems (1) an ein Anästhesiegasfortleitungssystem (50),
c. Einleiten des Atemgases (2) des Patienten in den Filter (20) des Systems über den Filtereinlass (21), wobei das Atemgas (2) ein Inhalationsanästhetikum aufweist,
d. Absaugen des mittels des Filters (20) gefilterten Atemgases (3) aus dem Pufferbehälterinnenraum (11) des Pufferbehälters (10) mittels des Anästhesiegasfortleitungssystems (50).

14. Verfahren nach Anspruch 13, wobei mittels eines Gassensors (40) eine Gasspezies, insbesondere das Inhalationsanästhetikum, in dem gefilterten Atemgas (3) detektiert wird.

15. Verfahren nach Anspruch 14, wobei mittels einer mit dem Gassensor (40) gekoppelten Hinweisvorrichtung (41) ein Hinweis erzeugt wird, wenn die Gasspezies von dem Gassensor (40) in dem gefilterten Atemgas (3) detektiert wird.
